# EUROPEAN PATENT APPLICATION

(11) **EP 4 385 536 A1**
(43) Date of publication of application: **19.06.2024**
(21) Application number: 22212704.5
(22) Date of filing: 12.12.2022
(51) Int. Cl.: A61M 1/06

(54) **BREAST SHIELD ASSEMBLY, ADJUSTMENT ARRANGEMENT, AND METHOD**

(71) Applicant: Koninklijke Philips N.V., 5656 AG Eindhoven (NL)
(72) Inventor: BOURQUIN, Yannyk Parulian Julian, Eindhoven (NL); VAN DER KOOI, Johannes Tseard, Eindhoven (NL); VAN DEN BIJGAART, Adrianus Wilhelmus Dionisius Maria, Eindhoven (NL); DAMODARAN, Mathivanan, Eindhoven (NL)
(74) Representative: Philips Intellectual Property & Standards

(57) **Abstract**

Provided is a breast shield assembly (112) for use in a breast manipulation procedure, for example milk expression and/or a breast massage procedure. The breast shield assembly comprises a cavity for receiving at least part of a breast, the cavity being at least partly defined by a surface (100) that faces the breast when the at least part of the breast is received in the cavity. The surface is arranged such that relative movement of the surface and the breast during the breast manipulation procedure causes the breast to be contactable by at least one location (111A, 111B) of the surface. An adjustment arrangement (110) enables varying of one or more of the at least one location such that different discrete regions of the breast are contactable by the surface. Further provided is an adjustment arrangement per se, a breast pump assembly comprising the breast shield assembly and a breast pump for moving the surface during the breast manipulation procedure, and a method of interfacing a breast with a surface of such a breast shield assembly.

## Description

### FIELD OF THE INVENTION

This invention relates to a breast shield assembly, a breast pump assembly including the breast shield assembly, and a method of interfacing a breast with a surface of such a breast shield assembly. The invention further relates to an adjustment arrangement for a breast shield.

### BACKGROUND OF THE INVENTION

Lactating women often suffer from sore nipples and blocked ducts: 69% suffer from blocked ducts, 97% suffer from sore nipples, and 20 % suffer from mastitis. When breastfeeding, mothers are advised to choose alternative positions to change the latch. A different latch gives a different mouth orientation that results in a different interaction with the breast/teat/nipple. This different interaction could open 'closed' ducts and avoid sore nipples. Both will result in less mastitis.

A further cause of sore nipples and blocked ducts arises from milk expression using a breast funnel and a breast pump. Whilst designs are known that aim to imitate the baby's mouth by having a collapsible breast funnel that touches the nipple. It is often the case that not all the milk ducts are drained. Furthermore, repetitive movements of the breast funnel against the nipple can result in stress spots on the nipple skin.

### SUMMARY OF THE INVENTION

The invention is defined by the claims.

According to examples in accordance with an aspect of the invention, there is provided a breast shield assembly for use in a breast manipulation procedure, the breast shield assembly comprising: a cavity for receiving at least part of a breast, the cavity being at least partly defined by a surface that faces the breast during use, wherein the surface is arranged such that relative movement of the surface and the breast during the breast manipulation procedure causes the breast to be contactable by a least one location of the surface; and an adjustment arrangement configured to enable varying one or more of the at least one location such that different discrete regions of the breast are contactable by the surface.

In spite of milk expression-related advantages associated with distinct location(s) of the surface contacting discrete region(s) of the breast, e.g. nipple and/or areola, repetitive contacting of the same discrete region(s) during milk expression can be undesirable because it can lead to only certain milk duct(s) being stimulated, in other words the milk duct(s) at or proximal to the discrete region(s), while other milk ducts do not receive such stimulation. The milk ducts not receiving stimulation risk becoming blocked, and the same discrete region(s) being repetitively contacted by the surface may be exposed to more friction. The latter can be a cause of nipple soreness.

To alleviate one or more of these problems, the configuration of the adjustment arrangement enables varying between different locations of the surface being contactable with the breast. In this manner, different discrete regions of the breast are contactable by the surface.

This can alleviate nipple soreness associated with the same discrete region(s) being repetitively contacted by the surface, and can reduce the risk of blocked milk duct(s) because the adjustment can result in different milk duct(s) being stimulated.

Moreover, since this is achieved via the adjustment arrangement included in the breast shield assembly, an orientation of one or more components of the breast shield assembly with respect to the breast can be maintained in spite of the varying of the at least one location. The capability to maintain this orientation can have various advantages.

In embodiments in which the breast shield assembly includes a milk receiving assembly for receiving milk during the milk expression, an orientation of the milk receiving assembly during milk expression may be independent of the varying of the location(s) of the surface that is or are contactable with the breast.

Thus, the varying of the location(s) of the surface that is or are contactable with the breast may not interfere with receiving of the expressed milk via the milk receiving assembly, e.g. collecting of milk in a collection container included in the milk receiving assembly. This may be beneficial particularly in the scenario that the orientation of the milk receiving assembly enables receiving of the expressed milk partly by gravity.

The adjustment arrangement can be configured in any suitable manner provided that the location(s) of the surface that is or are contactable with the breast can be changed so that different discrete regions of the breast are contactable by the surface.

In some embodiments, the adjustment arrangement is configured to enable the varying of one or more of the at least one location by moving position(s) of the surface towards the breast and/or away from the breast. Such movement may, for example, comprise radial movement of the position(s) towards and/or away from a central axis that extends through an opening of the cavity, with the breast being receivable in the cavity via movement along the central axis.

In some embodiments, the breast shield assembly comprises a wall that has a wall surface that at least partly defines the cavity.

In a first set of embodiments, the wall is a rigid wall. In such embodiments, the breast may be moveable towards and away from the rigid wall by a variable pressure provided in the cavity.

When the wall is such a rigid wall, the breast shield assembly may include an insert moveably, e.g. rotatably, mounted within the cavity, with movement, e.g. rotation, of the insert enabling a different region of the breast to be contacted by the insert when the breast is moved in the cavity, e.g. due to the above-mentioned variable pressure.

Thus, the location(s) of the surface in such embodiments include(s), e.g. is or are defined by, breast-facing surface(s) of the insert, with the adjustment arrangement comprising a mechanism, e.g. pivot coupling, that permits the breast-facing surface(s) to be moved so that different regions of the breast are enabled to contact the breast-facing surface(s).

In a second set of embodiments, the surface is at least partly included in a deformable wall, with the surface being moveable by deformation of the wall and recovery of a shape of the wall following the deformation.

In such embodiments, the adjustment arrangement may be configured to enable the varying of one or more of the at least one location by varying at least one axis along which the wall deforms or recovers.

In some embodiments, the cavity has a non-rotationally symmetric shape when the surface is not being moved, with the adjustment arrangement being configured to change a position and/or orientation of at least one portion of the wall that translates and/or rotates the non-rotationally symmetric cavity.

In some embodiments, and irrespective of whether or not the wall is deformable, the adjustment arrangement is provided at one or more of the following locations: around the wall; on an exterior surface of the wall, the exterior surface facing away from the breast during use; inside the wall; on the surface (whose location(s) are contactable with the breast); and within the cavity.

In some embodiments, a mechanical property of one or more portions of the deformable wall is variable.

For example, a stiffness of the deformable wall may be variable.

In such embodiments, the adjustment arrangement can be regarded as comprising a buckling mechanism, e.g. a fluidic or mechanical buckling mechanism, that implements said varying of the location(s) of the surface that is or are contactable with the breast.

For example the buckling mechanism may be configured to cause deformation/flexing of the wall that changes in successive pumping cycles so that first location(s) of the surface is or are brought into contact with first discrete region(s) of the breast followed by second location(s) of the surface being brought into contact with second discrete region(s) of the breast that is or are different from the first discrete region(s).

In some embodiments, the adjustment arrangement comprises, e.g. is in the form of, one or more springs, that is or are arranged such that application of stress thereto makes the spring(s) buckle towards an exterior surface of the deformable wall and thereby cause a pressure to be applied by the surface to the breast.

In other embodiments, the adjustment arrangement comprises, e.g. is in the form of, a piezoelectric buckling mechanism with a stack of at least two piezoelectric elements connected together.

In such embodiments, a relatively small piezoelectric displacement may result in a relatively large buckling displacement.

In some embodiments, the adjustment arrangement is at least partly defined by the wall comprising one or more non-Newtonian fluid portions that include(s) a non-Newtonian fluid, e.g. rubber, whose movement is responsive to the frequency of pressure variation provided by operation of a breast pump. In such embodiments, the pump frequency may be adjusted to enable the varying of the location(s) of the surface that is or are contactable with the breast.

In some embodiments, the adjustment arrangement comprises, e.g. is in the form of, at least one tube filled with non-Newtonian fluid. The at least one tube is attached to the flexible/deformable wall but may be provided around only a fraction of the deformable wall, e.g. about one third of the circumference of the deformable wall. When using a lower pumping frequency, the tube(s) may deform with the rest of the deformable wall to bring the location(s) of the surface of the deformable wall into contact with the breast. When applying a high frequency, the tube(s) may act stiffer than the deformable wall and cause it to deform in a different manner.

Alternatively or additionally, the adjustment arrangement may include one or more inflatable air cushions whose inflation and/or deflation enable(s) selection of the discrete region(s) of the breast contacted by the surface.

Alternatively or additionally, the adjustment arrangement may include an electroactive polymer assembly whose electroactive polymer(s) is or are deformable to enable the varying of the location(s) of the surface that is or are contactable with the breast.

By changing the voltage across electrodes between which the electroactive polymer(s) is or are disposed, the shape of the electroactive polymer assembly may be changed, with concomitant adjustment of the location(s) of the surface that is or are contactable with the breast.

In such embodiments, the surface may be at least partly included in the electroactive polymer(s).

Alternatively or additionally, the adjustment arrangement may include a heat change material assembly configured such that the surface is moveable in response to a thermal stimulus to vary the location(s) of the surface that is or are contactable with the breast.

In summary, the adjustment arrangement may comprise at least one of: an electroactive polymer assembly, at least one inflatable and/or deflatable air chamber, a piezoelectric buckling mechanism; a heat change material assembly; and one of more non-Newtonian fluid portions of the wall.

Alternatively or additionally, the adjustment arrangement comprises a wall-surrounding member configured to engage the wall to cause the at least one location to be contactable with the breast, the wall-surrounding member being moveable relative to the wall to enable said varying of one or more of the at least one location.

In such embodiments, the engagement of the exterior surface of the wall by the wall-surrounding member may determine a shape of the surface.

This shaping of the surface may provide location(s) that is or are flexed, e.g. inwardly collapsed, by the wall-surrounding member more than other regions. The greater flexing of the former may mean that they are more susceptible to flexing, e.g. inwardly collapsing, than the latter, in response to a variable pressure provided by operation of a breast pump. Thus, the more flexed, e.g. inwardly collapsed, location(s) may correspond to the location(s) that is or are contactable with the breast during the breast manipulation procedure.

In alternative embodiments, the wall-surrounding member may be cooperable with the deformable wall to control the resting state of the wall without impeding expansion of the cavity when a vacuum is applied. In such embodiments, the breast shield assembly may include a support structure comprising a rigid base to which the deformable wall, e.g. a rim of the deformable wall, is fixed, and a rigid wall-surrounding member arranged proximal to the rigid base. Since the rigid wall-surrounding member is close to the rigid base, expansion of the cavity may not, or may be only minimally, impeded by the wall-surrounding member.

Thus, the wall-surrounding member may enable changing of a position and/or orientation of at least one portion of the wall that translates and/or rotates the non-rotationally symmetric cavity.

In some embodiments, the wall-surrounding member is rotatable relative to the wall to permit the varying of one or more of the at least one location. This rotation of the wall-surrounding member relative to the wall may enable straightforward stimulation of different milk ducts, due to milk ducts being radially arranged around the nipple.

In some embodiments, the adjustment arrangement is manually operable by a user to enable said varying of one or more of the at least one location.

Such manual operability of the adjustment arrangement may provide a relatively simple and low-cost way for the user to control the breast shield assembly to enhance comfort and/or milk expression.

In some embodiments, the adjustment arrangement is manually operable by the user while the breast manipulation procedure, e.g. milk expression, is taking place. Alternatively or additionally, the adjustment arrangement is manually operable by the user to enable the varying of one or more of the at least one location prior to the breast manipulation procedure, e.g. milk expression, taking place.

In some embodiments, the adjustment arrangement, e.g. the wall-surrounding member, comprises a user-contactable portion configured to enable the user to manually move the adjustment arrangement, e.g. the wall-surrounding member, to enable the varying of one or more of the at least one location.

The user-contactable portion can be of any suitable type. In some embodiments, the user-contactable portion comprises a finger grip portion provided around an exterior surface of the wall-surrounding member, which exterior surface faces away from the surface.

In embodiments in which the adjustment arrangement, e.g. the wall-surrounding member, is rotatable, the user-contactable portion, e.g. finger grip portion, may facilitate manual rotation of the adjustment arrangement to permit the varying of one or more of the at least one location.

As an alternative or in addition to the above-described user adjustment of the adjustment arrangement, the adjustment arrangement may include a powered device controllable to implement the varying of one or more of the at least one location.

The powered device can be of any suitable type. In some embodiments, the powered device comprises one or more actuators, such as the above-described electroactive polymer assembly configured to move the surface in response to an electrical signal, and/or the above-described heat change material assembly configured to move the surface in response to a thermal stimulus.

In some embodiments, the adjustment arrangement comprises an automatic adjustment system configured to implement the varying of one or more of the at least one location.

Such an automatic adjustment system may, for example, automatically implement the varying based on pre-selected or pre-determined timings during which different discrete regions are being contacted by the surface, and/or based on milk collection data, e.g. collected via one or more sensors.

Such milk collection data may be indicative of when it might be beneficial, e.g. due to milk being collected at a slower rate, to implement the varying of one or more of the at least one location.

The automatic adjustment system may, for example, include one or more processors configured to control the powered device, and optionally configured to receive milk collection data from one or more sensors and control the powered device based on the received milk collection data.

According to another aspect there is provided a breast pump assembly comprising: the breast shield assembly according to any of the embodiments described herein; and a breast pump for providing a variable pressure that causes the relative movement of the surface and the breast during the breast manipulation procedure.

In some embodiments, the breast pump is a wearable breast pump.

In such embodiments, an orientation of the wearable breast pump during milk expression may be independent of the varying of one or more of the at least one location.

Thus, the varying of one or more of the at least one location may not interfere with the orientation of the wearable breast pump, hence the wearable breast pump can continue to be worn in the same manner during the varying of one or more of the at least one location.

According to yet another aspect there is provided an adjustment arrangement for a breast shield whose breast-receiving cavity is at least partly defined by a surface, the surface and the breast being moveable relative to each other during a breast manipulation procedure such that the breast is contactable by at least one location of the surface, wherein the adjustment arrangement is cooperable with the surface and configured to enable varying one or more of the at least one location such that different discrete regions of the breast are contactable by the surface.

In some embodiments, the adjustment arrangement, for example the adjustment arrangement comprising the wall-surrounding member, may be retrofittable to the breast shield.

In the case of the adjustment arrangement comprising, e.g. being, the wall-surrounding member, the wall surrounding member may be a ring for arranging around the deformable wall. Such a ring may, for instance, comprise two (or more) ring portions that can be positioned around the wall and coupled to each other to define the wall-surrounding member.

According to a further aspect there is provided a method of interfacing a breast with a surface of a breast shield assembly suitable for use in a breast manipulation procedure, the breast being contactable by at least one location of the surface during relative movement of the surface and the breast in the breast manipulation procedure, the breast shield assembly further comprising an adjustment arrangement, the method comprising using the adjustment arrangement to vary one or more of the at least one location such that different discrete regions of the breast are contactable by the surface.

The breast shield assembly may be according to any of the embodiments described herein.

In some embodiments, the method does not include the breast manipulation procedure, for example does not include expression of milk from the breast to which the surface is interfaced.

The method may set the breast shield assembly into a state, i.e. with the surface being interfaced with the breast and the at least one location(s) being selected to make contact with the breast, which permits subsequent implementing of the breast manipulation procedure, e.g. milk expression, but without the breast manipulation procedure, e.g. milk expression, being included in the claimed method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a perspective view of part of a breast shield assembly according to an example;
FIGs. 2A to 2E provide various views of a breast shield assembly according to an example;
FIG. 3 shows a first example of an adjustment arrangement of a breast shield assembly;
FIG. 4 shows a second example of an adjustment arrangement of a breast shield assembly;
FIG. 5 shows a third example of an adjustment arrangement of a breast shield assembly;
FIG. 6 shows a breast shield assembly according to another example; and
FIGs. 7A and 7B depict two positions of an adjustment arrangement included in the breast shield assembly shown in FIG. 6.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a breast shield assembly for use in a breast manipulation procedure, for example milk expression and/or a breast massage procedure. The breast shield assembly comprises a cavity for receiving at least part of a breast, the cavity being at least partly defined by a surface that faces the breast when the at least part of the breast is received in the cavity. The surface is arranged such that relative movement of the surface and the breast during the breast manipulation procedure causes the breast to be contactable by at least one location of the surface. An adjustment arrangement enables varying of one or more of the at least one location such that different discrete regions of the breast are contactable by the surface. Further provided is an adjustment arrangement per se, a breast pump assembly comprising the breast shield assembly and a breast pump for moving the surface during the breast manipulation procedure, and a method of interfacing a breast with a surface of such a breast shield assembly.

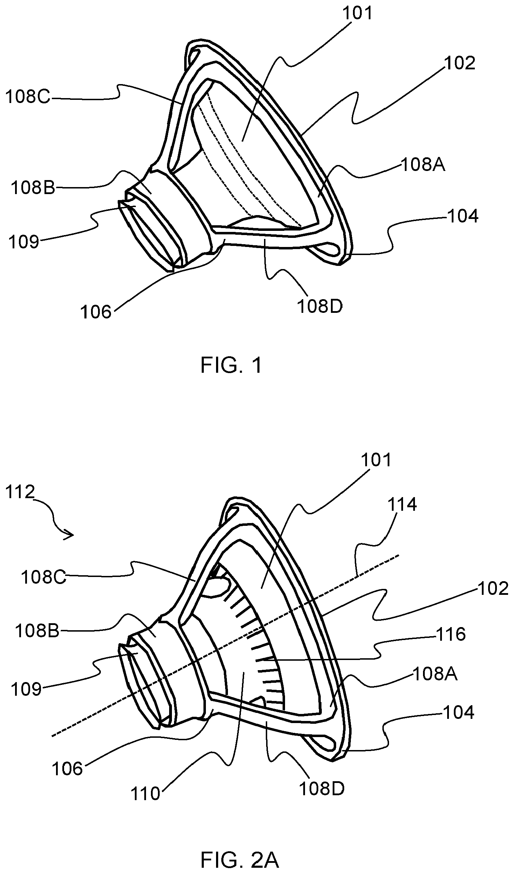

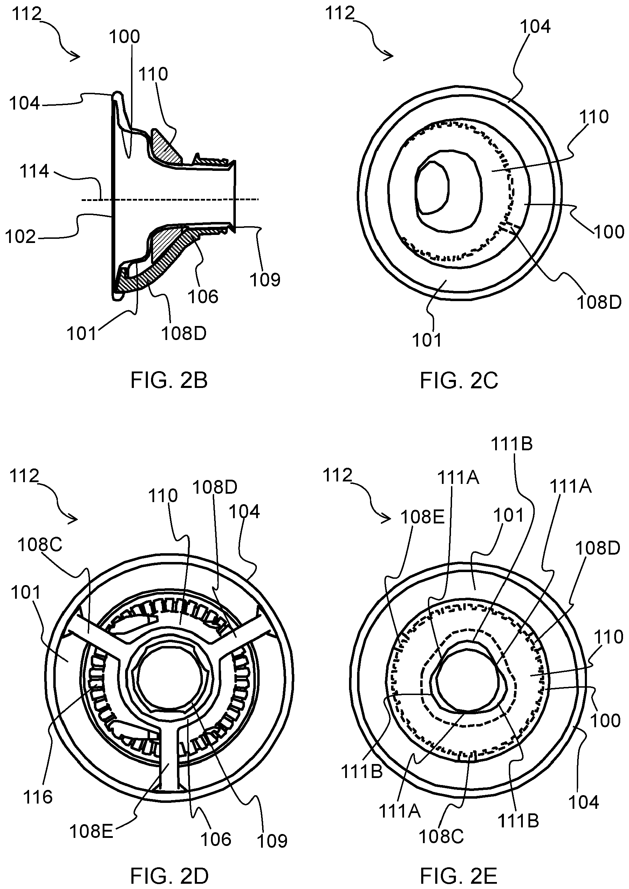

FIG. 1 shows a breast shield having a wall 101 whose surface 100 (see FIGs. 2B, 2C and 2E) at least partly defines a cavity for receiving at least part of a breast, e.g. a nipple-comprising region of a breast (not visible in FIGs. 1 and 2A to 2E). The surface 100 together with the at least part of the breast may at least partly define a chamber in which a variable pressure can be provided during a breast manipulation procedure, e.g. a breast manipulation procedure comprising expressing milk from the breast and/or a breast massage procedure.

Such a variable pressure can be provided by operation of a breast pump (not visible).

In some embodiments, and as shown in FIG. 1, the surface 100 is included in a wall 101, such as in a deformable wall 101 that defines a flexible funnel in which the nipple-comprising region of the breast is receivable. The term "flexible" can be regarded as meaning that the flexible funnel is resiliently deformable.

The surface 100 may be at least partly defined by an inner surface of the flexible funnel that, in use, faces the breast.

In such embodiments, the flexible funnel may delimit an open mouth 102 for receiving at least the nipple of the breast.

The open mouth 102 may be delimited by a rim 104 of the flexible funnel that extends at least partly around the nipple-comprising region of the breast.

Deformation/flexing of the flexible funnel or recovery of the flexible funnel following deformation/flexing may generate a mechanical pressure applied to the nipple-comprising region of the breast, as will be explained in more detail herein below.

In some embodiments, such flexing and recovery of the flexible funnel also creates a variable vacuum in the chamber that facilitates expression of milk from the breast.

The wall 101 may be formed of any suitable material. In embodiments in which the wall 101 is deformable, the wall 101 is made of flexible, in other word resiliently deformable, materials.

In some embodiments, the wall 101, e.g. the flexible funnel, is formed from an elastomeric material, such as silicone rubber.

In some embodiments, and as shown in FIG. 1, the flexible funnel is supported by a support structure 106. Such a support structure 106 may support the flexible funnel during flexing and subsequent recovery of the flexible funnel.

The support structure 106 may have any suitable design provided that the support structure 106 is capable of supporting the flexible funnel during flexing and recovery of the flexible funnel.

In some embodiments, and as shown in FIG. 1, the support structure 106 comprises a mouth-supporting portion 108A that extends at least partly around and supports the open mouth 102 of the flexible funnel, and a base-supporting portion 108B that extends at least partly around a base 109 of the funnel-shaped wall 101, which base 109 opposes the open mouth 102.

In such embodiments, the support structure 106 may include one or more support ribs 108C, 108D that extend between the mouth-supporting portion 108A and the base-supporting portion 108B. Whilst two of the support ribs 108C, 108D are visible in FIG. 1, the support structure 106 in this non-limiting example includes a third support rib 108E which can be seen, for instance, in FIGs. 2D and 2E.

More generally, and referring to FIGs. 2A to 2E, a breast shield assembly 112 includes an adjustment arrangement 110 that enables varying of one or more location(s) 111A, 111B of the surface 100 that contact(s) the breast during the breast manipulation procedure such that different discrete regions of the breast are contactable by the surface 100.

For example, the adjustment arrangement 110 may cooperate with the surface 100 to enable varying of one or more of the location(s) 111A, 111B of the surface 100 that contact(s) the breast.

In spite of milk expression-related advantages associated with distinct, e.g. discrete, location(s) 111A, 111B of the surface 100 contacting discrete region(s) of the breast, e.g. nipple and/or areola, repetitive contacting of the same discrete region(s) during the breast manipulation procedure, e.g. during milk expression, can be undesirable because it can lead to only certain milk duct(s) being stimulated, in other words the milk duct(s) at or proximal to the discrete region(s), while other milk ducts do not receive such stimulation. The milk ducts not receiving stimulation risk becoming blocked, and the same discrete region(s) being repetitively contacted by the surface 100 may be exposed to more friction. The latter can be a cause of nipple soreness.

To alleviate these problems, the adjustment arrangement 110 enables varying, e.g. changing between, different locations 111A, 111B of the surface 100 being contactable with the breast. In this manner, different discrete regions of the breast are contactable by the surface 100.

This can alleviate nipple soreness associated with the same discrete region(s) being repetitively contacted by the surface 100, and can reduce the risk of blocked milk duct(s) because the adjustment can result in different milk duct(s) being stimulated.

Moreover, since this is achieved via the adjustment arrangement 110 included in the breast shield assembly, an orientation of one or more components of the breast shield assembly 112, such as the flexible funnel, with respect to the breast can be maintained during the varying. The capability to maintain this orientation can have various advantages.

In some embodiments, the positioning of the rim 104 of the wall 101, e.g. the flexible funnel, that extends at least partly around the at least part of the breast, e.g. and is, in use, arranged against the breast, may be independent of the varying.

Hence the positioning of the wall 101, e.g. the flexible funnel, against the breast need not be altered by the adjustment, thereby making the breast shield assembly 112 more convenient to use.

Alternatively or additionally, in embodiments in which the breast shield assembly 112 includes a milk receiving assembly (not visible) for receiving milk during the milk expression, an orientation of the milk receiving assembly during milk expression may be independent of the varying of one or more of the at least one location 111A, 111B.

Thus, the varying of one or more of the at least one location 111A, 111B may not interfere with receiving of the expressed milk via the milk receiving assembly, e.g. collecting of milk in a collection container included in the milk receiving assembly. This may be beneficial particularly in the scenario that the orientation of the milk receiving assembly enables receiving of the expressed milk partly by gravity.

Alternatively or additionally, in embodiments in which the breast shield assembly 112 includes the support structure 106 for supporting the wall 101, e.g. the flexible funnel, during deformation of the wall 101, an orientation of the support structure 106 relative to the breast may be independent of the varying of one or more of the at least one location 111A, 111B.

The adjustment arrangement 110 can be configured in any suitable manner provided that the location(s) 111A, 111B of the surface 100 that is or are contactable with the breast can be changed so that different discrete regions of the breast are contactable by the surface 100.

In some embodiments, such as that shown in FIGs. 2A to 2E, the adjustment arrangement 110 is configured to enable the varying of one or more of the at least one location 111A, 111B by moving position(s) of the surface 100 towards the breast and/or away from the breast. Such movement may, for example, comprise radial movement of the position(s) 111A, 111B towards and/or away from a central axis 114 that extends through the open mouth 102 of the cavity, with the breast being receivable in the cavity via movement along the central axis 114.

In some embodiments, the adjustment arrangement 110 comprises a buckling mechanism, e.g. a fluidic or mechanical buckling mechanism, that implements said varying of the location(s) 111A, 111B of the surface 100 that is or are contactable with the breast.

In such embodiments, the buckling mechanism may be configured to cause flexing of the flexible funnel that changes in successive pumping cycles so that first location(s) 111A is or are brought into contact with the first discrete region(s) of the breast followed by second location(s) 111B being brought into contact with second discrete region(s) of the breast that is or are different from the first discrete region(s).

In some embodiments, the adjustment arrangement 110 comprises, e.g. is in the form of, one or more springs, that is or are arranged such that application of stress thereto makes the spring(s) buckle towards an exterior surface of the deformable wall 101 and thereby cause a pressure to be applied by the surface 100 to the breast.

In other embodiments, the adjustment arrangement comprises, e.g. is in the form of, a piezoelectric buckling mechanism with a stack of at least two piezoelectric elements connected together.

In such embodiments, a relatively small piezoelectric displacement may result in a relatively large buckling displacement.

In some embodiments, the adjustment arrangement 110 is at least partly defined by the wall 101 comprising one or more non-Newtonian fluid portions that include(s) a non-Newtonian fluid, e.g. rubber, whose movement is responsive to the frequency of pressure variation provided by operation of a breast pump. In such embodiments, the pump frequency may be adjusted to enable the varying of the location(s) 111A, 111B of the surface 100 that is or are contactable with the breast.

For example, the adjustment arrangement 110 comprises a non-Newtonian fluid, e.g. rubber, whose movement is responsive to the frequency of pressure variation provided by operation of the breast pump. In such embodiments, the pump frequency may be adjusted to enable switching between the first location(s) 111A being contactable with the first discrete region(s) of the breast and the second location(s) 111B being contactable with the second discrete region(s) of the breast.

In some embodiments, the adjustment arrangement 110 comprises, e.g. is in the form of, at least one tube filled with non-Newtonian fluid. The at least one tube is attached to the flexible/deformable wall 101 but may be provided around only a fraction of the deformable wall 101, e.g. about one third of the circumference of the deformable wall 101. When using a lower pumping frequency, the tube(s) may deform with the rest of the deformable wall 101 to bring the location(s) 111A, 111B of the surface 100 of the deformable wall 101 into contact with the breast. When applying a high frequency, the tube(s) may act stiffer than the deformable wall 101 and cause it to deform in a different manner.

Alternatively or additionally, the adjustment arrangement 110 may include one or more inflatable air cushions whose inflation and/or deflation enable(s) selection of the discrete region(s) of the breast contacted by the surface 100.

Alternatively or additionally, the adjustment arrangement 110 may include an electroactive polymer assembly whose electroactive polymer(s) is or are deformable to enable the varying of the location(s) 111A, 111B of the surface 100 that is or are contactable with the breast.

By changing the voltage across electrodes between which the electroactive polymer(s) is or are disposed, the shape of the electroactive polymer assembly may be changed, with concomitant adjustment of the location(s) 111A, 111B of the surface 100 that is or are contactable with the breast.

In such embodiments, the surface 100 may be at least partly included in the electroactive polymer(s).

Alternatively or additionally, the adjustment arrangement 110 may include a heat change material assembly configured such that the surface 100 is moveable in response to a thermal stimulus to vary the location(s) 111A, 111B of the surface 100 that is or are contactable with the breast.

In some embodiments, and referring again to FIGs. 2A to 2E, the wall 101, e.g. the flexible funnel, and the adjustment arrangement 110 are configured to cooperate such that flexing of the wall 101 causes either first location(s) 111A or second location(s) 111B of the surface 100 to contact respective discrete region(s) of the breast.

The adjustment arrangement 110 may control deformation, e.g. collapse, of the wall 101 so as to cause or force the surface 100 to deform in order to enable the varying of the location(s) 111A, 111B of the surface 100 that is or are contactable with the breast.

In some embodiments, such as that shown in FIGs. 2A to 2E, the adjustment arrangement 110 is configured to enable varying, e.g. selection, of at least one axis along which the wall 101, e.g. the flexible funnel, deforms, e.g. collapses, towards the nipple of the breast.

In some embodiments, the adjustment arrangement 110 is moveable relative to the surface 100 to enable the varying of the location(s) 111A, 111B of the surface 100 that is or are contactable with the breast.

This may provide a relatively straightforwardly implementable way of selecting the region(s) of the breast contactable by the surface 100.

In some embodiments, such as that shown in FIGs. 2A to 2E, the adjustment arrangement 110 is rotatable around the surface 100 to enable the varying of the location(s) 111A, 111B of the surface 100 that is or are contactable with the breast.

In such embodiments, at least part of the adjustment arrangement 110 may be rotatable about the central axis 114 of the breast shield assembly 112 that, in use, extends away from the nipple of the breast.

This may enable straightforward stimulation of different milk ducts, due to milk ducts being radially arranged around the nipple.

In some embodiments, such as that shown in FIGs. 2A to 2E, the adjustment arrangement 110 comprises a wall-surrounding member configured to engage the wall 101, e.g. an exterior surface of the wall 101 that, in use, faces away from the breast received in the cavity. Such engagement may cause the at least one location 111A, 111B to be contactable with the breast.

In such embodiments, the engagement of the exterior surface of the deformable wall 101, e.g. the flexible funnel, by the wall-surrounding member may determine a shape of the surface 100 of the wall, e.g. the flexible funnel.

This shaping of the surface of the wall 101, e.g. the flexible funnel, may provide portion(s) that is or are flexed, e.g. inwardly collapsed, by the wall-surrounding member more than other portions. The greater flexing of the former may mean they are more susceptible to flexing, e.g. inward collapsing, than the latter, in response to a variable pressure provided by operation of a breast pump.

Thus, the more flexed, e.g. inwardly collapsed, portions may correspond to the first location(s) 111A or the second location(s) 111B depending on adjustment of the cooperation between the adjustment arrangement 110, in particular the wall-surrounding member, and the surface 100.

In some embodiments, such as that shown in FIGs. 2A to 2E, the wall-surrounding member, included in the adjustment arrangement 110, is rotatable relative to the wall 101, e.g. the flexible funnel, to permit the varying of the location(s) 111A, 111B of the surface 100 that is or are contactable with the breast. Thus, rotation of the wall-surrounding member may enable switching between the at least one first location 111A being contactable with first discrete region(s) of the breast and the at least one second location 111B being contactable with second discrete region(s) of the breast. This rotation of the wall-surrounding member relative to the wall 101, e.g. the flexible funnel, may enable straightforward stimulation of different milk ducts, due to milk ducts being radially arranged around the nipple.

Whilst the embodiments described above have involved a deformable wall 101, e.g. defining a flexible funnel, this is not intended to be limiting, and in other embodiments the wall 101 may be a rigid wall 101. In such embodiments, the breast may be moveable towards and away from the rigid wall 101 by a variable pressure provided in the cavity, e.g. a variable pressure provided by a breast pump.

When the wall 101 is such a rigid wall 101, the breast shield assembly 112 may include an insert moveably, e.g. rotatably, mounted within the cavity, with movement, e.g. rotation, of the insert enabling a different region of the breast to be contacted by the insert when the breast is moved in the cavity, e.g. due to the above-mentioned variable pressure.

Thus, the location(s) 111A, 111B of the surface 100 in such embodiments include(s), e.g. is or are defined by, breast-facing surface(s) of the insert, with the adjustment arrangement comprising a mechanism, e.g. pivot coupling, that permits the breast-facing surface(s) to be moved so that different regions of the breast are enabled to contact the breast-facing surface(s).

More generally, the adjustment arrangement 110 may be manually operable by a user to enable the varying of the location(s) 111A, 111B of the surface 100 that is or are contactable with the breast.

Such manual operability of the adjustment arrangement 110 may provide a relatively simple and low-cost way for the user to control the breast shield assembly 112 to enhance comfort and/or milk expression.

This may, for instance, involve manually operating the adjustment arrangement 110 so as to switch to the surface 100 contacting one or more discrete regions proximal to milk duct(s) less stimulated in a previous breast manipulation procedure, e.g. pumping session, and/or to avoid one or more discrete regions corresponding to a sore area of the breast.

In some embodiments, the adjustment arrangement 110 is manually operable by the user while the breast manipulation procedure, e.g. milk expression, is taking place.

Alternatively or additionally, the adjustment arrangement 110 is manually operable by the user to select the one or more discrete regions contacted by the surface 100 prior to milk expression taking place.

In some embodiments, and as shown in FIGs. 2A to 2E and 3 to 5, the adjustment arrangement 110, e.g. the wall-surrounding member, comprises a user-contactable portion 116 configured to enable the user to manually move the adjustment arrangement 110, e.g. the wall-surrounding member, to cause the varying of the location(s) 111A, 111B of the surface 100 that is or are contactable with the breast.

The user-contactable portion 116 can be of any suitable type. In some embodiments, the user-contactable portion 116 comprises a finger grip portion provided around an exterior surface of the adjustment arrangement 110, e.g. around an exterior surface of the wall-surrounding member, which exterior surface faces away from the surface 100.

In embodiments in which the adjustment arrangement 110, e.g. the wall-surrounding member, is rotatable, the user-contactable portion 116, e.g. finger grip portion, may facilitate manual rotation of the adjustment arrangement 110 to permit the varying of the location(s) 111A, 111B of the surface 100 that is or are contactable with the breast.

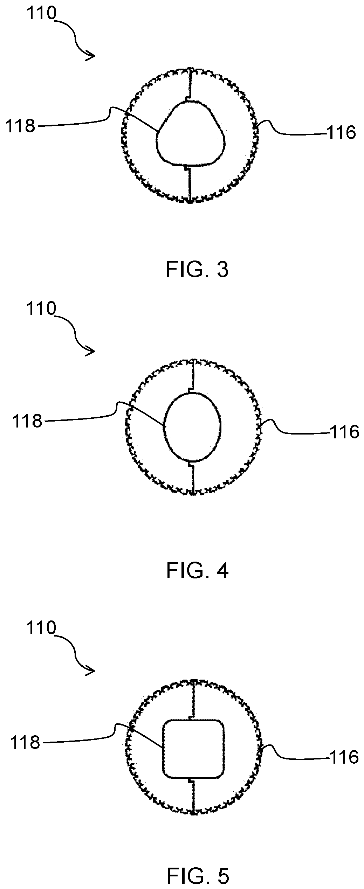

Various different designs for the adjustment arrangement 110, in particular the wall-surrounding member, are shown in FIGs. 3 to 5. In the design shown in FIG. 3, the wall-surrounding member is cooperable with the wall 101, in this case the flexible funnel, so that the respective distinct locations 111A, 111B each have three defined locations contactable with the breast. This is due to the three-lobed epitrochoid shape of the aperture 118 of the wall-surrounding member that engages the exterior surface of the wall 101.

In the design shown in FIG. 4, the wall-surrounding member is cooperable with the wall 101, in this case the flexible funnel, so that the respective distinct locations 111A, 111B each have two defined locations contactable with the breast. This is due to the ellipse or oval shape of the aperture 118 of the wall-surrounding member that engages the exterior surface of the wall 101.

In the design shown in FIG. 5, the wall-surrounding member is cooperable with the wall 101, in this case the flexible funnel, so that the respective distinct locations 111A, 111B each have four defined locations contactable with the breast. This is due to the four-lobed epitrochoid shape of the aperture 118 of the wall-surrounding member that engages the exterior surface of the wall 101.

More generally, any number of defined locations contactable with the breast, for each selectable distinct location(s) 111A, 111B, can be contemplated, such as one, two, three, four, five, six, seven, eight, nine, or ten.

In a specific non-limiting example, the adjustment arrangement 110 comprises a wall-surrounding member, e.g. an adjustment ring, with an oval aperture (see FIG. 4), through which part of the wall 101 in the form of a flexible funnel is passed. The oval shape of the aperture in this illustrative example slightly changes a resting state of the flexible funnel and forces the flexible funnel to collapse along a specific axis, e.g. along a specific radial axis towards the central axis 114. By rotating the wall-surrounding member relative to the flexible funnel, the specific axis along which the funnel collapses can be controlled, in other words selected. In this way, the design enables the varying of the one or more of the at least one location 111A, 111B such that different discrete regions of the breast are contactable with the surface 100.

A prototype according to this specific non-limiting example was built and tested by placing the flexible funnel on a dummy breast and recording videos of the collapse of the flexible funnel from the interior of the flexible funnel.

In this case, the resting state of the flexible funnel already had an oval shape as the wall-surrounding member pressed slightly on the flexible funnel to give it this shape. The collapse of the flexible membrane was observed at only two corners defined by the wall-surrounding member. When the wall-surrounding member was rotated relative to the flexible funnel, the position of the corners was shifted and thus the collapse happened with the two corners in the shifted position.

In this manner, adjustment of the cooperation between the wall-surrounding member and the flexible funnel was observed to enable switching from two first locations 111A being respectively contactable with two first discrete region(s) of the breast and two second locations 111B being respectively contactable with two second discrete region(s) of the breast.

For comparison, a similar video was made for a flexible funnel without any adjustment arrangement 110 (see FIG. 1). In this case, the flexible funnel collapsed such that four corners of the flexible funnel made contact with the nipple of the dummy breast, and reverted to a recovered, round, state after release of the vacuum. During the whole video, the collapse of the membrane was always in the same fashion with the four corners at the same position. This is illustrative of the above-described repetitive contacting of the same discrete region(s) during the breast manipulation procedure, e.g. milk expression.

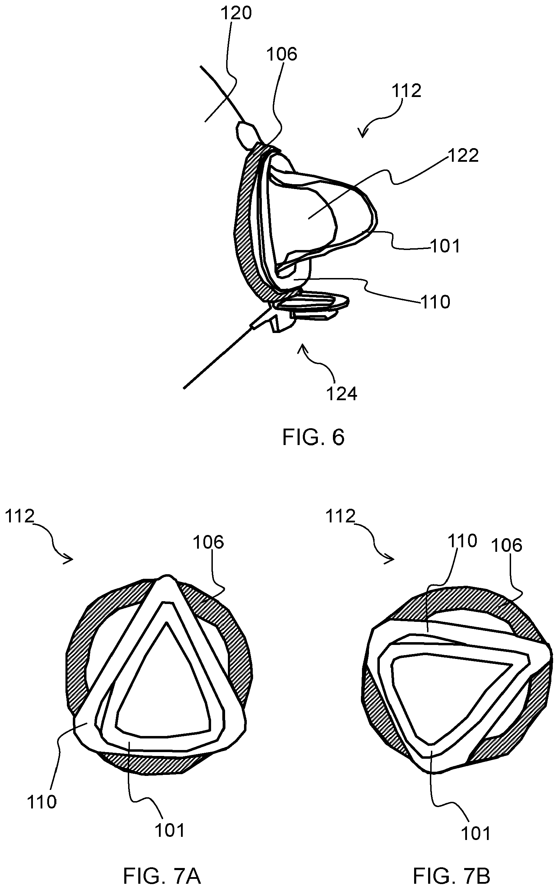

In some embodiments, such as that shown in FIGs. 6, 7A and 7B, the cavity has a non-rotationally symmetric shape when the surface 100 is not being moved, with the adjustment arrangement 110 being configured to change a position and/or orientation of at least one portion of the wall 101, e.g. flexible funnel, that translates and/or rotates the non-rotationally symmetric cavity.

In such embodiments, the deformable wall 101, e.g. flexible funnel, and the adjustment arrangement 110 may be configured to cooperate such that recovery of the wall 101 following flexing causes the at least one location 111A, 111B to contact the breast 120.

In such embodiments, the deformable wall 101, e.g. the flexible funnel, may be regarded as being in a collapsed resting state when no vacuum is applied, and may expand when a vacuum is applied.

Hence the adjustment arrangement 110 may be cooperable with the flexible funnel to control the resting state of the flexible funnel without impeding expansion of the flexible funnel when a vacuum is applied.

To this end, the breast shield assembly 112 may include a support structure 106 comprising a rigid base to which the flexible funnel, e.g. the rim 104 of the flexible funnel, is fixed, and an adjustment arrangement 110 comprising a rigid wall-surrounding member arranged proximal to the rigid base. Since the rigid wall-surrounding member is close to the rigid base, expansion of the flexible funnel may not, or may be only minimally, impeded by the wall-surrounding member. The rigid wall-surrounding member may be rotatable to obtain different deformations of the flexible funnel, as illustrated in FIGs. 7A and 7B.

Another possibility would be to arrange the wall-surrounding member to cover a nipple portion of the flexible funnel and fabricate the wall-surrounding member from a flexible material that is stiff enough to deform the flexible funnel but flexible enough to expand during the expansion of the flexible funnel.

More generally in such embodiments, the adjustment arrangement 110 is adjustable to permit selection of at least one axis along which the deformable wall 101, e.g. flexible funnel, recovers towards the nipple 122 of the breast 120.

Selection of the at least one axis may permit the varying of the location(s) 111A, 111B of the surface 100 that is or are contactable with the breast 120, as previously described in relation to the embodiments shown in FIGs. 2A to 5.

FIG. 6 shows an embodiment in which the breast shield assembly 112 includes a milk receiving assembly 124 for receiving milk during the milk expression. In such embodiments, an orientation of the milk receiving assembly during milk expression may be independent of the configuration of the adjustment arrangement 110 to vary the location(s) 111A, 111B of the surface 100 that is or are contactable with the breast 120, as previously described.

In some embodiments, such as those shown in FIGs. 2A to 2E, 6, 7A and 7B, the adjustment arrangement 110 is manually operable by a user to vary the location(s) 111A, 111B of the surface 100 that is or are contactable with the breast 120. Thus, the user may manually adjust the adjustment arrangement 110 according to their preference, in particular to increase comfort and/or enhance milk expression via stimulation of different milk ducts.

As an alternative or in addition to the above-described user adjustment of the adjustment arrangement 110, the adjustment arrangement 110 may include a powered device (not visible) controllable to implement the varying of the location(s) 111A, 111B of the surface 100 that is or are contactable with the breast 120.

The powered device can be of any suitable type. In some embodiments, the powered device comprises one or more actuators, such as the above-described electroactive polymer assembly configured to move the surface 100 in response to an electrical signal, and/or the above-described heat change material assembly configured to move the surface 100 in response to a thermal stimulus.

Alternatively or additionally, the adjustment arrangement 110, e.g. the powered device included in the adjustment arrangement 110, may include an inflation device controllable to inflate and/or deflate the above-described one or more inflatable air cushions whose inflation and/or deflation enable(s) the varying of the location(s) 111A, 111B of the surface 100 that is or are contactable with the breast 120.

More generally, in some embodiments the adjustment arrangement 110 comprises an automatic adjustment system configured to implement the varying of one or more of the at least one location 111A, 111B.

Such an automatic adjustment system may, for example, automatically implement the varying based on pre-selected or pre-determined timings during which different discrete regions are being contacted by the surface 100, and/or based on milk collection data, e.g. collected via one or more sensors.

Such milk collection data may be indicative of when it might be beneficial, e.g. due to milk being collected at a slower rate, to implement the varying of one or more of the at least one location 111A, 111B.

The automatic adjustment system may, for example, include one or more processors configured to control the powered device, and optionally configured to receive milk collection data from one or more sensors and control the powered device based on the received milk collection data.

Further provided is a breast pump assembly comprising the breast shield assembly 112 according to any of the embodiments described herein, and a breast pump for providing a variable pressure that causes the relative movement of the surface 100 and the breast 120 during the breast manipulation procedure.

Thus, the main elements of this aspect may be the breast pump, the breast shield assembly 112, for example comprising the wall 101, e.g. a funnel with a collapsible material, and the adjustment arrangement 110.

For example, the adjustment arrangement 110 may include a controllable funnel collapse part that allows control of the collapsible funnel, and optionally a user interface, e.g. the user-contactable portion 116, to allow the user to control the controllable funnel collapse part.

In some embodiments, the breast pump is a wearable breast pump.

In such embodiments, an orientation of the wearable breast pump during milk expression may be independent of the varying of one or more of the at least one location 111A, 111B.

Thus, the varying of one or more of the at least one location 111A, 111B may not interfere with the orientation of the wearable breast pump, hence the wearable breast pump can continue to be worn in the same manner during the varying of one or more of the at least one location 111A, 111B.

Further provided is an adjustment arrangement 110 for a breast shield whose breast-receiving cavity is at least partly defined by a surface 100, the surface 100 and the breast 120 being moveable relative to each other during a breast manipulation procedure such that the breast 120 is contactable by at least one location 111A, 111B of the surface 100, wherein the adjustment arrangement 110 is cooperable with the surface 100, e.g. by being cooperable with a deformable wall 101 that includes at least part of the surface 100, and is configured to enable varying one or more of the at least one location 111A, 111B such that different discrete regions of the breast 120 are contactable by the surface 100.

In some embodiments, the adjustment arrangement 110, for example the adjustment arrangement 110 comprising the wall-surrounding member, may be retrofittable to the breast shield.

In the case of the adjustment arrangement 110 comprising, e.g. being, the wall-surrounding member, the wall-surrounding member may be a ring for arranging around the deformable wall 101 of the breast shield. Such a ring may, for instance, comprise two (or more) ring portions that can be positioned around the wall 101 and coupled to each other to define the wall-surrounding member.

Such couplable ring portions may enable retrofitting of the wall-surrounding member in spite of the inclusion of the support rib(s) 108C, 108D, 108E described above in relation to FIGs. 1 and 2A to 2E.

Evident in FIGs. 3 to 5 are the joins between the ring portions at which the ring portions, two half-ring portions in this case, are couplable to each other.

Further provided is a method of interfacing a breast 120 with a surface 100 of a breast shield assembly 112 suitable for use in a breast manipulation procedure. The breast 120 is contactable by at least one location 111A, 111B of the surface 100 during relative movement of the surface 100 and the breast 120 in the breast manipulation procedure. The breast shield assembly 112 further comprises an adjustment arrangement 110. The breast shield assembly 112 can be according to any of the embodiments described herein.

The method comprises using the adjustment arrangement 110 to vary one or more of the at least one location 111A, 111B such that different discrete regions of the breast are contactable by the surface 100.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A breast shield assembly (112) for use in a breast manipulation procedure, the breast shield assembly comprising:
a cavity for receiving at least part of a breast, the cavity being at least partly defined by a surface (100) that faces the breast during use, wherein the surface is arranged such that relative movement of the surface and the breast during the breast manipulation procedure causes the breast to be contactable by at least one location (111A, 111B) of the surface; and
an adjustment arrangement (110) configured to enable varying one or more of the at least one location such that different discrete regions of the breast are contactable by the surface.

2. The breast shield assembly (112) according to claim 1, wherein the adjustment arrangement (110) is configured to enable said varying of one or more of the at least one location (111A, 111B) by moving position(s) of the surface (100) towards the breast and/or by moving position(s) of the surface away from the breast.

3. The breast shield assembly (112) according to claim 1 or claim 2, comprising a wall (101) that includes at least part of the surface (100), the wall being deformable, the surface being moveable by deformation of the wall and recovery of a shape of the wall following said deformation.

4. The breast shield assembly (112) according to claim 3, wherein the adjustment arrangement (110) is configured to enable said varying of one or more of the at least one location (111A, 111B) by varying at least one axis along which the wall (101) deforms or recovers.

5. The breast shield assembly (112) according to claim 3 or claim 4, wherein the cavity has a non-rotationally symmetric shape when the surface (100) is not being moved, the adjustment arrangement (110) being configured to change a position and/or orientation of at least one portion of the wall (101) that translates and/or rotates the non-rotationally symmetric cavity.

6. The breast shield assembly (112) according to any one of claims 3 to 5, wherein the adjustment arrangement (110) is provided at one or more of the following locations:
around the wall (101);
on an exterior surface of the wall, the exterior surface facing away from the breast during use;
inside the wall;
on said surface (100); and
within the cavity.

7. The breast shield assembly (112) according to any one of claims 3 to 6, wherein a mechanical property of one or more portions of the wall (101) is variable.

8. The breast shield assembly (112) according to any one of claims 3 to 7, wherein the adjustment arrangement (110) comprises at least one of: an electroactive polymer assembly, at least one inflatable and/or deflatable air chamber, a piezoelectric buckling mechanism; a heat change material assembly; and one of more non-Newtonian fluid portions of the wall (101).

9. The breast shield assembly (112) according to any one of claims 3 to 8, wherein the adjustment arrangement (110) comprises a wall-surrounding member configured to engage the wall (101) to cause the at least one location (111A, 111B) to be contactable with the breast, the wall-surrounding member being moveable relative to the wall to enable said varying of one or more of the at least one location.

10. The breast shield assembly (112) according to claim 9, wherein the wall-surrounding member is rotatable relative to the wall (101) to enable said varying of one or more of the at least one location (111A, 111B).

11. The breast shield assembly (112) according to any one of claims 1 to 10, wherein the adjustment arrangement (110) is manually operable by a user to enable said varying of one or more of the at least one location (111A, 111B).

12. The breast shield assembly (112) according to any one of claims 1 to 11, wherein the adjustment arrangement (110) comprises a powered device controllable to implement the varying of one or more of the at least one location (111A, 111B).

13. A breast pump assembly for use in a breast manipulation procedure, the breast pump assembly comprising:
the breast shield assembly (112) according to any of claims 1 to 12; and
a breast pump for providing a variable pressure that causes the relative movement of the surface (100) and the breast during the breast manipulation procedure.

14. An adjustment arrangement (110) for a breast shield whose breast-receiving cavity is at least partly defined by a surface, the surface and the breast being moveable relative to each other during a breast manipulation procedure such that the breast is contactable by at least one location of the surface, wherein the adjustment arrangement is cooperable with the surface and configured to enable varying one or more of the at least one location such that different discrete regions of the breast are contactable by the surface.

15. A method of interfacing a breast with a surface (100) of a breast shield assembly (112) suitable for use in a breast manipulation procedure, the breast being contactable by at least one location of the surface during relative movement of the surface and the breast in the breast manipulation procedure, the breast shield assembly further comprising an adjustment arrangement (110), the method comprising using the adjustment arrangement to vary one or more of the at least one location such that different discrete regions of the breast are contactable by the surface.
